# EUROPEAN PATENT APPLICATION

(11) **EP 4 825 812 A1**
(43) Date of publication of application: **21.10.2026**
(21) Application number: 24903759.9
(22) Date of filing: 12.12.2024
(51) Int. Cl.: C02F 1/24, C07C 51/64, C07C 53/21

(54) **FOAM SEPARATION TREATMENT METHOD, AQUEOUS SOLUTION, METHOD FOR USING VINYL ALCOHOL-BASED POLYMER, AND FOAM GENERATION PROMOTER**

(30) Priority: 13.12.2023 JP 2023209810
(71) Applicant: Kuraray Co., Ltd., Kurashiki-shi, Okayama 710-0801 (JP)
(72) Inventor: NII, Shinsuke, Kurashiki-shi, Okayama 713-8550 (JP)
(74) Representative: Müller-Boré & Partner Patentanwälte PartG mbB
(86) International application number: PCT/JP2024/044024
(87) International publication number: WO 2025/127106

(57) **Abstract**

A foam separation treatment method of separating an organofluorine compound from water to be treated by collecting a foam generated by feeding a gas into the water to be treated containing the organofluorine compound, wherein the foam separation treatment method comprises introducing a polymer surfactant into the water to be treated. This provides a foam separation treatment method by which an organofluorine compound can be efficiently separated from water to be treated containing the organofluorine compound.

## Description

### Technical Field

The present disclosure relates to a foam separation treatment method, an aqueous solution, a method of using a vinyl alcohol-based polymer, and a foam generation promoter.

### Background Art

In recent years, environmental pollution problems due to perfluorooctanesulfonic acid (PFOS) and perfluorooctanoic acid (PFOA) classified as organofluorine compounds (PFAS) have become apparent, and a technique of separating and recovering these chemical substances (Patent Literature 1) and a technique of decomposing these chemical substances (Patent Literature 2) have been proposed. Among the techniques of separating and recovering chemical substances, a water treatment method called a foam separation method is known, and Patent Literature 1 discloses a foam separation treatment apparatus to be used in the foam separation method.

### Citation List

### Patent Literature

Patent Literature 1: JP 2023-50657 A
Patent Literature 2: JP 2022-537895 A

### Summary of Invention

### Technical Problem

In separating and recovering PFAS in water to be treated by a foam separation treatment, when the water to be treated exhibits poor foaming, the efficiency of foam separation may be poor.

An object of the present disclosure is to provide a foam separation treatment method by which PFAS can be efficiently separated from water to be treated containing PFAS, an aqueous solution, a method of using a vinyl alcohol-based polymer, and a foam generation promoter.

### Solution to Problem

The above object is achieved by providing any of the following:
[1] a foam separation treatment method of separating an organofluorine compound from water to be treated by collecting a foam generated by feeding a gas into the water to be treated containing the organofluorine compound, wherein the foam separation treatment method comprises introducing a polymer surfactant into the water to be treated;
[2] the foam separation treatment method according to claim 1, wherein the polymer surfactant is a vinyl alcohol-based polymer;
[3] the foam separation treatment method according to [2], wherein the vinyl alcohol-based polymer has a viscosity-average degree of polymerization of 100 or more and 5,000 or less and a degree of saponification of 30 mol% or more and 99.99 mol% or less;
[4] the foam separation treatment method according to any one of [1] to [3], wherein the polymer surfactant is introduced into the water to be treated such that a content of the polymer surfactant in the water to be treated is 1% by mass or less;
[5] the foam separation treatment method according to any one of [1] to [4], wherein the polymer surfactant is introduced in an amount of 1 or more times by mass an amount of the organofluorine compound contained in the water to be treated;
[6] an aqueous solution comprising a vinyl alcohol-based polymer and an organofluorine compound;
[7] the aqueous solution according to [6], wherein the vinyl alcohol-based polymer has a viscosity-average degree of polymerization of 100 or more and 5,000 or less and a degree of saponification of 30 mol% or more and 99.99 mol% or less;
[8] the aqueous solution according to [6] or [7], wherein a content of the vinyl alcohol-based polymer in the aqueous solution is 1% by mass or less;
[9] the aqueous solution according to any one of [6] to [8], wherein a content of the vinyl alcohol-based polymer is 1 or more times by mass a content of the organofluorine compound in the aqueous solution;
[10] a foam separation treatment method comprising: feeding a gas into the aqueous solution according to any one of [6] to [9] to generate a foam; and collecting the foam;
[11] a method of using a vinyl alcohol-based polymer in a treatment of separating an organofluorine compound from water to be treated by collecting a foam generated by feeding a gas into the water to be treated containing the organofluorine compound,
   the method comprising mixing a vinyl alcohol-based polymer with the water to be treated to promote generation of the foam;
[12] a foam generation promoter comprising a vinyl alcohol-based polymer to be introduced into water to be treated when an organofluorine compound is separated from the water to be treated by a foam separation treatment.

### Advantageous Effects of Invention

According to the foam separation treatment method, the aqueous solution, the method of using a vinyl alcohol-based polymer, and the foam generation promoter of the present disclosure, the separation efficiency of PFAS can be improved. This makes it possible to efficiently separate an organofluorine compound from water to be treated, and is useful for purifying water contaminated with an organofluorine compound.

### Description of Embodiments

### <Foam separation treatment method>

The foam separation treatment method of the present disclosure is a foam separation treatment method of separating an organofluorine compound from water to be treated by collecting a foam generated by feeding a gas into the water to be treated containing the organofluorine compound, wherein the foam separation treatment method comprises introducing a polymer surfactant into the water to be treated.

In the foam separation treatment method of the present disclosure, a polymer surfactant may be introduced into water to be treated containing an organofluorine compound (PFAS), a gas may be fed into the water to be treated to generate bubbles, the polymer surfactant and PFAS may be concentrated at an air-water interface of the bubbles to generate a foam, and the foam may be collected in the vicinity of the surface of the water to be treated.

The method of introducing the polymer surfactant into the water to be treated is not particularly limited as long as the objective of the present invention is not impaired. For example, the polymer surfactant may be added to the water to be treated before the gas is fed into the water to be treated, or the polymer surfactant may be added to the water to be treated while the gas is fed into the water to be treated. In addition, for example, the water to be treated may be charged into a treatment tank or the like with the polymer surfactant charged therein in advance, followed by the execution of a foam separation treatment.

The gas fed into the water to be treated is not particularly limited, but for example, air, nitrogen, or the like is used, and air is preferable.

Means for feeding the gas into the water to be treated is not particularly limited as long as it can generate a foam, and for example, the gas may be fed through an air pipe installed in the water to be treated.

After the foam separation treatment, the collected mixture of the polymer surfactant and PFAS may be incinerated as it is, or may be adsorbed on an adsorbent such as activated carbon and then incinerated.

In one embodiment of the foam separation treatment method of the present disclosure, the foam separation treatment may be continuously conducted, and for example, at least one step among a step of feeding the water to be treated into an apparatus for the foam separation treatment, a step of introducing the polymer surfactant into the water to be treated, a step of feeding the gas into the water to be treated, and a step of collecting the generated foam may be continuously conducted.

The foam separation treatment according to the foam separation treatment method of the present disclosure may be a treatment for separating and recovering a part of PFAS contained in the water to be treated to reduce the concentration of PFAS in the water to be treated, or a treatment for separating and recovering substantially the entire amount of PFAS contained in the water to be treated to remove PFAS from the water to be treated.

The amount of the polymer surfactant to be introduced into the water to be treated is not particularly limited as long as the objective of the present invention is not impaired, but the content of the polymer surfactant in the water to be treated is preferably 0.1 ppm by mass or more, more preferably 1 ppm by mass or more, still more preferably 10 ppm by mass or more, and, in some cases, further preferably 100 ppm by mass or more. The content of the polymer surfactant in the water to be treated is preferably 1% by mass or less. That is, in the foam separation treatment method of the present disclosure, it is preferable to introduce the polymer surfactant into the water to be treated such that the content of the polymer surfactant in the water to be treated falls within the above range. When the content of the polymer surfactant in the water to be treated is within the above range, further improved foamability is achieved. In one embodiment, even when the foam separation treatment is continuously conducted, it is preferable to adjust the amount of the polymer surfactant to be introduced into the water to be treated such that the content of the polymer surfactant in the water to be treated falls within the above range.

The amount of the polymer surfactant is preferably 1 or more times by mass, more preferably 10 or more times by mass, still more preferably 50 or more times by mass, and further preferably 100 or more times by mass the content of PFAS in the water to be treated. That is, in the foam separation treatment method of the present disclosure, it is preferable to introduce the polymer surfactant in an amount of 1 or more times by mass the amount of PFAS contained in the water to be treated. When the amount of the polymer surfactant relative to the content of PFAS in the water to be treated is within the above range, the recovery efficiency of PFAS is enhanced more. In one embodiment, even when the foam separation treatment is continuously conducted, it is preferable to adjust the introduction amount of the water to be treated into the treatment system and/or the amount of the polymer surfactant to be introduced into the water to be treated such that the amount of the polymer surfactant relative to the content of PFAS in the water to be treated falls within the above range.

The water to be treated may contain components such as other impurities in addition to PFAS as long as the objective of the present invention is not impaired.

### (Polymer surfactant)

The polymer surfactant in the present disclosure is a compound that is a polymer and exhibits a surface-active action. The phrase "being a polymer" means having a molecular weight of 1000 or more. In the foam separation treatment method of the present disclosure, one polymer surfactant may be used, or two or more polymer surfactants may be used.

The molecular weight of the polymer surfactant is 1000 or more, preferably 2000 or more, more preferably 5000 or more, and still more preferably 10000 or more.

The polymer surfactant preferably has an HLB value of 3 or more, more preferably 8 or more, and still more preferably 13 or more. The HLB value is an index indicating the degree of affinity of the surfactant for water and oil, and takes a value from 0 to 20. The closer to 0 the HLB value is, the higher the lipophilicity of the surfactant is, whereas the closer to 20 the HLB value is, the higher the hydrophilicity of the surfactant is. Herein, the HLB value is a value defined by the Griffin method, and HLB value = 20 × (sum of formula weights of hydrophilic moieties)/(molecular weight). For example, when a surfactant is strongly mixed in water, the case where a part of the surfactant is dispersed in the water corresponds to an HLB value of the surfactant of 3 to 6; the case where the surfactant is dispersed in a milky state corresponds to an HLB value of 6 to 8; the case where the surfactant is dispersed in a stable milky state corresponds to an HLB value of 8 to 10; the case where a translucent or transparent dispersion is formed corresponds to an HLB value of 10 to 13; and the case where the surfactant is dissolved and becomes transparent corresponds to an HLB value of 13 or more.

The polymer surfactant may be a polymer obtained by polymerizing monomers, and is preferably a polymer of a nonionic monomer and/or an anionic monomer. Examples of the nonionic monomer include ethylene oxide, propylene oxide, (meth)acrylamide-based compounds, alkyl (meth)acrylates, styrene, acrylonitrile, and vinyl acetate. Here, examples of the alkyl (meth)acrylates include methyl (meth)acrylate, ethyl (meth)acrylate, butyl (meth)acrylate, and hydroxyethyl (meth)acrylate. Examples of the anionic monomer include (meth)acrylic acid, vinylsulfonic acid, 2-acrylamide-2-methylpropanesulfonic acid, maleic acid, and salts thereof. In the present disclosure, "(meth)acryl" is a collective term for methacryl and acryl. The polymer surfactant may be a polymer obtained by polymerizing one kind of monomer, or a polymer obtained by copolymerizing two or more kinds of monomers. In addition, the polymer surfactant may be a polymer obtained through, for example, a step of introducing a functional group after polymerization of a monomer. Among polymer surfactants, a vinyl alcohol-based polymer (PVA) obtained from a vinyl ester-based monomer such as vinyl acetate as a raw material is preferable because it is further superior in water solubility, foam formability, and biodegradability. That is, a preferred aspect of the foam separation treatment method of the present disclosure is a foam separation treatment method of separating an organofluorine compound from water to be treated containing the organofluorine compound by collecting a foam generated by feeding a gas into the water to be treated, in which PVA is introduced into the water to be treated. The monomer as a raw material of PVA may be a vinyl ester-based monomer other than vinyl acetate as described later.

PVA is a polymer having a vinyl alcohol unit as a monomer unit. The PVA is obtained by saponifying a vinyl ester-based polymer obtained by polymerizing a vinyl ester-based monomer as a raw material monomer thereof, and the PVA after saponification may contain a vinyl ester unit in addition to the vinyl alcohol unit.

In addition, the PVA may be a PVA obtained by saponifying a copolymer obtained by copolymerizing a vinyl ester-based monomer as a raw material monomer with other monomers to obtain a PVA containing a monomer unit other than the vinyl alcohol unit and the vinyl ester unit, or may be a PVA obtained by introducing a specific functional group into a side chain or a terminal by reacting a specific chemical species in a polymerization step, in a saponification step, and/or after saponification in a PVA production process. In the present disclosure, such PVA may be referred to as "modified vinyl alcohol-based polymer (modified PVA)". In the present disclosure, an agent to be used to modify PVA may be referred to as a "modifier". For example, in one embodiment, when the modified PVA has a terminal structure derived from a chain transfer agent, the chain transfer agent may be referred to as a modifier, and in another embodiment, when the modified PVA is a product formed by copolymerizing a vinyl ester-based monomer with "other monomers" other than the vinyl ester-based monomer, the "other monomers" may be referred to as a modifier. In the present disclosure, the proportion of units derived from a modifier to all monomer units constituting a modified PVA may be referred to as a "modification amount".

The lower limit of the proportion of the vinyl alcohol unit to all monomer units in the PVA is preferably 35 mol%, more preferably 50 mol%, and, in some cases, still more preferably 65 mol%. On the other hand, the upper limit of the proportion of the vinyl alcohol unit is preferably 99.95 mol%, more preferably 95 mol%, and, in some cases, still more preferably 90 mol%.

The viscosity-average degree of polymerization of the PVA is preferably 100 or more and 5,000 or less. The lower limit of the viscosity-average degree of polymerization of the PVA is more preferably 200, and may be still more preferably 500. The upper limit of the viscosity-average degree of polymerization of the PVA is more preferably 4,000, still more preferably 3,000, and may be further preferably 2,000. When the viscosity-average degree of polymerization is within the above range, production tends to be easier. The viscosity-average degree of polymerization is a value obtained by measurement in accordance with JIS K 6726:1994. Specifically, when the degree of saponification of a PVA is less than 99.5 mol%, the viscosity-average degree of polymerization of a PVA saponified until the degree of saponification becomes 99.5 mol% or more is obtained by the following formula using the limiting viscosity [η] (liter/g) measured at 30°C in water. $P = {\left(\left[η\right]\times {10}^{4}/8.29\right)}^{\left(1/0.62\right)}$

The lower limit of the degree of saponification of the PVA is preferably 30 mol%, more preferably 50 mol%, and may be still more preferably 65 mol%. On the other hand, the upper limit of the degree of saponification of the PVA is preferably 99.99 mol%, more preferably 99 mol%, still more preferably 90 mol%, and may be further preferably 80 mol%. The degree of saponification of the PVA is preferably 30 mol% or more and 99.99 mol% or less, more preferably 50 mol% or more and 99.99 mol% or less, and may be still more preferably 65 mol% or more and 90 mol% or less. By setting the degree of saponification of the PVA within the above range, the efficiency of foam separation tends to be improved, and the PVA tends to be industrially more stably produced.

The method for producing the PVA preferably includes a polymerization step of polymerizing a vinyl ester-based monomer and a saponification step of saponifying the vinyl ester-based polymer obtained thereby.

The vinyl ester-based polymer can be produced by polymerizing a vinyl ester-based monomer by a conventionally known method such as a bulk polymerization method, a solution polymerization method, a suspension polymerization method, an emulsion polymerization method, or a dispersion polymerization method. From the viewpoint of enhancing the effect of the present disclosure, a solution polymerization method in which polymerization is conducted using a lower alcohol is preferable. The lower alcohol is not particularly limited, but an alcohol having 3 or less carbon atoms such as methanol, ethanol, propanol, or isopropanol is preferable, and methanol is more preferable. As the polymerization operation, any polymerization method of a batch method, a semi-batch method, and a continuous method can be employed.

Examples of the vinyl ester-based monomer include vinyl formate, vinyl acetate, vinyl propionate, vinyl butyrate, vinyl isobutyrate, vinyl pivalate, vinyl versatate, vinyl caproate, vinyl caprylate, vinyl laurate, vinyl palmitate, vinyl stearate, vinyl oleate, and vinyl benzoate. Among them, vinyl acetate is preferable.

As described above, the PVA may be a modified PVA obtained by copolymerizing a vinyl ester-based monomer with other monomers within a range where the objective of the present disclosure is not impaired. Examples of the other monomers include α-olefins such as ethylene, propylene, n-butene, and isobutylene; (meth)acrylic acid and salts thereof; (meth)acrylic acid esters such as methyl (meth)acrylate, ethyl (meth)acrylate, n-propyl (meth)acrylate, i-propyl (meth)acrylate, n-butyl (meth)acrylate, i-butyl (meth)acrylate, t-butyl (meth)acrylate, 2-ethylhexyl (meth)acrylate, dodecyl (meth)acrylate, and octadecyl (meth)acrylate; acrylamide-based compounds such as (meth)acrylamide, N-methyl(meth)acrylamide, N-ethyl(meth)acrylamide, N,N-dimethyl(meth)acrylamide, diacetone (meth)acrylamide, (meth)acrylamidopropanesulfonic acid and salts thereof, (meth)acrylamidopropyldimethylamine and salts or quaternary salts thereof, N-methylol(meth)acrylamide and derivatives thereof; vinyl ethers such as methyl vinyl ether, ethyl vinyl ether, n-propyl vinyl ether, i-propyl vinyl ether, n-butyl vinyl ether, i-butyl vinyl ether, t-butyl vinyl ether, dodecyl vinyl ether, and stearyl vinyl ether; nitriles such as acrylonitrile and methacrylonitrile; vinyl halides such as vinyl chloride and vinyl fluoride; vinylidene halides such as vinylidene chloride and vinylidene fluoride; allyl compounds such as allyl acetate and allyl chloride; unsaturated dicarboxylic acids such as maleic acid, itaconic acid, and fumaric acid, and salts or esters thereof; and isopropenyl acetate. These can be used alone or two or more thereof can be used in combination. Among them, 3-(methacryloylamino)propyltrimethylammonium chloride (MAPTAC) is preferable as the acrylamide-based compounds. The copolymerization amount (modification amount) of other monomers is preferably 10 mol% or less. The copolymerization amount of other monomers means a proportion of a unit derived from other monomers to all monomer units constituting the modified PVA.

The polymerization initiator to be used in the polymerization can be selected from among known initiators (for example, an azo initiator, a peroxide initiator, and a redox initiator). Examples of the azo initiator include 2,2'-azobisisobutyronitrile, 2,2'-azobis(2,4-dimethylvaleronitrile), and 2,2'-azobis(4-methoxy-2,4-dimethylvaleronitrile). Examples of the peroxide initiator include percarbonate compounds such as di-n-propyl peroxydicarbonate, diisopropyl peroxydicarbonate, di-2-ethylhexyl peroxydicarbonate, and diethoxyethyl peroxydicarbonate; perester compounds such as t-butyl peroxyneodecanoate, α-cumyl peroxyneodecanoate, and t-butyl peroxydecanoate; acetylcyclohexylsulfonyl peroxide; and 2,4,4-trimethylpentyl-2-peroxyphenoxyacetate. Potassium persulfate, ammonium persulfate, hydrogen peroxide, and the like may be combined with those initiators to form initiators. Examples of the redox initiator include initiators composed of the above peroxides combined with reducing agents such as sodium hydrogen sulfite, sodium hydrogen carbonate, tartaric acid, L-ascorbic acid, and Rongalite. When the polymerization is conducted at a high temperature, coloring due to decomposition of the vinyl ester-based monomer may be observed. In that case, for the purpose of preventing the coloring, an antioxidant such as tartaric acid may be added to the polymerization system in an amount of about 1 to 100 ppm by mass relative to the vinyl ester-based monomer.

In the polymerization of the vinyl ester-based monomer, a chain transfer agent may coexist for the purpose of adjusting the degree of polymerization of the resulting PVA, introducing a functional group into the PVA, or the like. Examples of the chain transfer agent include aldehydes such as acetaldehyde, propionaldehyde, butyraldehyde, and benzaldehyde; ketones such as acetone, methyl ethyl ketone, hexanone, and cyclohexanone; mercaptans such as 2-hydroxyethanethiol; thiocarboxylic acids such as thioacetic acid; halogenated hydrocarbons such as trichloroethylene and perchloroethylene, and alkyl mercaptans such as normal dodecyl mercaptan (n-DDM), and among them, aldehydes and alkyl mercaptans are suitably used. The addition amount of the chain transfer agent is determined according to the chain transfer constant of the chain transfer agent to be added and the degree of polymerization of the target PVA, and is generally preferably 0.1 to 10% by mass relative to the vinyl ester to be used. As one embodiment, a modified PVA in which an alkyl group derived from an alkyl mercaptan is introduced at a terminal can be obtained by using an alkyl mercaptan as a chain transfer agent in the polymerization step of PVA. The modification amount of such a modified PVA, that is, the proportion of the unit having an alkyl group derived from an alkyl mercaptan to all the monomer units constituting the modified PVA is preferably 0.01 mol% or more and 5 mol% or less, more preferably 0.1 mol% or more and 3 mol% or less, still more preferably 0.3 mol% or more and 1 mol% or less. n-DDM is preferable as the alkyl mercaptan.

The polymerization temperature is not particularly limited, and is preferably 0 to 180°C, more preferably 20 to 160°C, and still more preferably 30 to 150°C. When the polymerization is conducted at a temperature equal to or lower than the boiling point of the solvent used in the polymerization step, either reduced pressure boiling polymerization in which polymerization is conducted while boiling the solvent under reduced pressure, or normal pressure non-boiling polymerization in which polymerization is conducted under such a condition that the solvent is not boiled under normal pressure can be selected. When polymerization is conducted at a temperature equal to or higher than the boiling point of the solvent used in the polymerization step, either pressure non-boiling polymerization in which polymerization is conducted under such a condition that the solvent is not boiled under pressure, or pressure boiling polymerization in which polymerization is conducted while boiling the solvent under pressure can be selected.

As described above, the PVA is obtained by saponifying a vinyl ester-based polymer. Examples of the method of the saponification reaction include an alcoholysis reaction or a hydrolysis reaction using a basic catalyst such as sodium hydroxide, potassium hydroxide, or sodium methoxide, an acidic catalyst such as p-toluenesulfonic acid, or the like. Examples of the solvent that can be used in this reaction include alcohols such as methanol and ethanol; esters such as methyl acetate and ethyl acetate; ketones such as acetone and methyl ethyl ketone; aromatic hydrocarbons such as benzene and toluene. These solvents may be used alone, or two or more of them may be used in combination. In particular, it is convenient and preferable to conduct saponification using methanol or a methanol/methyl acetate mixed solution as the solvent and sodium hydroxide as the catalyst.

### (Organofluorine compound)

The organofluorine compound (PFAS) may be an amphiphilic perfluoroalkane derivative, and examples of the amphiphilic perfluoroalkane derivative include perfluoroalkane carboxylic acid, perfluoroalkyl sulfonic acid, and 1H,1H,2H,2H-perfluoroalkyl alcohol. Examples of the perfluoroalkane carboxylic acid include perfluorobutanoic acid, perfluoropentanoic acid, perfluorohexanoic acid, perfluoroheptanoic acid, perfluorooctanoic acid, perfluorononanoic acid, perfluorodecanoic acid, perfluoroundecanoic acid, and perfluorododecanoic acid. In particular, the foam separation treatment method of the present disclosure is suitable for perfluoropentanoic acid, perfluorohexanoic acid, perfluoroheptanoic acid, perfluorooctanoic acid, and the like. Examples of the perfluoroalkyl sulfonic acid include perfluorobutane sulfonic acid, perfluorohexane sulfonic acid, perfluorooctane sulfonic acid, and salts thereof. Examples of the 1H,1H,2H,2H-perfluoroalkyl alcohol include 1H,1H,2H,2H-perfluoro-1-hexanol, 1H,1H,2H,2H-perfluoro-1-octanol, and 1H,1H,2H,2H-perfluoro-1-decanol. The PFAS to be subjected to the foam separation treatment may be either one kind or two or more kinds.

### <Aqueous solution>

One embodiment of the present invention is an aqueous solution comprising PVA and PFAS. By this composition of the aqueous solution, the PFAS contained in the aqueous solution can be easily separated by the foam separation treatment.

Preferred aspects of the PVA and the PFAS contained in the aqueous solution are the same as those of the PVA and the PFAS described above.

The content of the PVA in the aqueous solution is preferably 0.1 ppm by mass or more, more preferably 1 ppm by mass or more, still more preferably 10 ppm by mass or more, and, in some cases, further preferably 100 ppm by mass or more. The content of the PVA in the aqueous solution is preferably 1% by mass or less. When the content of the PVA in the aqueous solution is within the above range, the aqueous solution is further superior in foamability.

The content of the PVA in the aqueous solution is preferably 1 or more times by mass, more preferably 10 or more times by mass, still more preferably 50 or more times by mass, and further preferably 100 or more times by mass relative the content of the PFAS. When the content of the PVA is within the above range, further superior PFAS recovery efficiency is achieved.

The aqueous solution of the present disclosure may contain various additives and impurities as long as the objective of the present invention is not impaired.

Further, one embodiment of the present invention is a foam separation treatment method in which a gas is fed into the above aqueous solution to generate a foam, and the foam is collected.

### <Method of using vinyl alcohol-based polymer>

One embodiment of the present invention is a method of using a PVA in a treatment of separating a PFAS from water to be treated by collecting a foam generated by feeding a gas into the water to be treated containing the PFAS, the method comprising mixing a PVA with the water to be treated to promote generation of the foam.

Preferred aspects of the PVA in the method of use are the same as those of the PVA described above.

### <Foam generation promoter>

One embodiment of the present invention is a foam generation promoter comprising a vinyl alcohol-based polymer to be introduced into water to be treated when an organofluorine compound is separated from the water to be treated by a foam separation treatment.

A preferred aspects of the PVA in the foam generation promoter are the same as those of the PVA described above.

### Examples

Hereafter, the present invention will be described in more detail with reference to examples, but the present invention is not limited to these examples at all. "%" and "ppm" in examples and comparative examples represent "% by mass" and "ppm by mass", respectively, unless otherwise specified.

### [Viscosity-average degree of polymerization]

The viscosity-average degree of polymerization of the PVAs used in Examples was measured in accordance with JIS K 6726:1994. Specifically, when the degree of saponification was less than 99.5 mol%, the viscosity-average degree of polymerization of a PVA saponified until the degree of saponification became 99.5 mol% or more was obtained by the following formula using the limiting viscosity [η] (liter/g) measured at 30°C in water.$P = {\left(\left[η\right]\times {10}^{4}/8.29\right)}^{\left(1/0.62\right)}$

### [Degree of saponification]

The degree of saponification of the PVAs used in Examples was measured in accordance with JIS K 6726:1994.

### <Production Example 1>

### [Production of PVA1]

PVA1 was produced as a polymer surfactant by the following method. A 3 L reactor equipped with a stirrer, a reflux condenser, a nitrogen inlet tube, and an initiator addition port was charged with 640 g of vinyl acetate and 960 g of methanol, and the inside of the system was purged with nitrogen for 30 minutes while nitrogen bubbling was conducted. Heating of the reactor was started, and at a time when the internal temperature reached 60°C, 0.3 g of 2,2'-azobisisobutyronitrile (AIBN) was added to initiate polymerization. After the polymerization was conducted at 60°C for 5 hours, 0.3 g of hydroquinone was added, and the mixture was cooled to stop the polymerization. The solid concentration at the time of stopping the polymerization was 20.0%, and the polymerization rate was 50%. Subsequently, the unreacted vinyl acetate monomer was removed at 50°C under a reduced pressure while methanol was sometimes added, affording a methanol solution (concentration: 35%) of a vinyl ester-based polymer (PVAc). Furthermore, methanol was added thereto to prepare a methanol solution of the vinyl ester-based polymer (the amount of the vinyl ester-based polymer in the solution: 160.0 g). To 457 g of the solution was added 3.7 g of an alkali solution (10% methanol solution of sodium hydroxide), and saponification was conducted (vinyl ester-based polymer concentration of saponification solution: 30%, molar ratio of sodium hydroxide to vinyl acetate units: 0.005). A gel-like material was formed about 15 minutes after the addition of the alkali solution, and this was pulverized with a pulverizer and left to stand at 40°C for 1 hour to advance the saponification. Then, 500 g of methyl acetate was added to neutralize the remaining alkali. After confirming the completion of the neutralization using a phenolphthalein indicator, the mixture was filtered to afford a white solid, to which 2,000 g of methanol was added, and the resulting mixture was left to stand at room temperature for 3 hours and washed. The above washing operation was repeated three times, and then the white solid obtained by centrifugal deliquoring was left to stand at 65°C for two days in a dryer, affording PVA1. PVA1 had a viscosity-average degree of polymerization of 530 and a degree of saponification of 88.0 mol%.

### <Production Examples 2 to 4>

### [Production of PVA2 to PVA4]

PVA2 to PVA4 were produced as polymer surfactants by the following method. PVA2 to PVA4 were obtained in the same manner as in the production of PVA1 in Production Example 1 except that the polymerization conditions (amount of vinyl acetate used, amount of methanol used, polymerization rate) and the saponification conditions (PVAc concentration, molar ratio of sodium hydroxide to PVAc) were changed as shown in Table 1.

### <Production Example 5>

### [Production of PVA5]

PVA5 was produced as a polymer surfactant by the following method. A 3 L reactor equipped with a stirrer, a reflux condenser, a nitrogen inlet tube, a delay solution dropping port, and an initiator addition port was charged with 960 g of vinyl acetate, 640 g of methanol, and 4.3 g of 3-(methacryloylamino)propyltrimethylammonium chloride (MAPTAC) as a comonomer, and the inside of the system was purged with nitrogen for 30 minutes while nitrogen bubbling was conducted. In addition, a comonomer solution containing MAPTAC dissolved at a concentration of 20% in methanol was prepared as a delay solution, and the comonomer solution was purged with nitrogen by nitrogen gas bubbling. Heating of the reactor was started, and at a time when the internal temperature reached 60°C, 0.2 g of 2,2'-azobisisobutyronitrile (AIBN) was added to initiate polymerization. While the monomer composition (ratio between vinyl acetate and MAPTAC) in the polymerization solution was kept constant by adding the delay solution dropwise, polymerization was conducted at 60°C for 3 hours. Then, 0.4 g of hydroquinone was added, and the mixture was cooled to stop the polymerization. The total amount of MAPTAC added until the polymerization was stopped was 13.4 g. The solid concentration at the time of stopping the polymerization was 17.0%, and the polymerization rate was 30%. Subsequently, the unreacted vinyl acetate monomer was removed at 50°C under a reduced pressure while methanol was sometimes added, affording a methanol solution (concentration: 35%) of a MAPTAC-modified vinyl ester-based polymer. Then, saponification was conducted under the same conditions as in Production Example 1 described above except that the saponification conditions (PVAc concentration, molar ratio of sodium hydroxide to PVAc) were changed as shown in Table 1, and PVA5 was thereby obtained. The modification amount (the proportion of units derived from MAPTAC to all monomer units constituting PVA5) was 2.0 mol%. The modification amount of PVA5 was determined by measuring the MAPTAC-modified vinyl ester-based polymer as a precursor before the saponification by ¹H-NMR.

### <Production Example 6>

### [Production of PVA6]

PVA6 was produced as a polymer surfactant by the following method. A 3 L reactor equipped with a stirrer, a reflux condenser, a nitrogen inlet tube, a delay solution dropping port, and an initiator addition port was charged with 960 g of vinyl acetate, 640 g of methanol, and 0.36 g of normal dodecyl mercaptan (n-DDM), and the inside of the system was purged with nitrogen for 30 minutes while nitrogen bubbling was conducted. In addition, a chain transfer agent solution containing n-DDM dissolved at a concentration of 10% in methanol was prepared as a delay solution, and the solution was purged with nitrogen by nitrogen gas bubbling. Heating of the reactor was started, and at a time when the internal temperature reached 60°C, 1.0 g of 2,2'-azobisisobutyronitrile (AIBN) was added to initiate polymerization. While the ratio between vinyl acetate and n-DDM in the polymerization solution was kept constant by adding the delay solution dropwise, polymerization was conducted at 60°C for 3 hours. Then, 2.0 g of hydroquinone was added, and the mixture was cooled to stop the polymerization. The total amount of n-DDM added until the polymerization was stopped was 6.32 g. The solid concentration at the time of stopping the polymerization was 28.9%, and the polymerization rate was 50%. Subsequently, the unreacted vinyl acetate monomer was removed at 50°C under a reduced pressure while methanol was sometimes added, affording a methanol solution (concentration: 35%) of a n-DDM-modified vinyl ester-based polymer. Then, saponification was conducted under the same conditions as in Production Example 1 described above, affording PVA6, which is a modified PVA having a structure derived from n-DDM at the terminal. The modification amount of PVA6 (the proportion of units having an alkyl group derived from n-DDM to all monomer units constituting PVA6) was 0.5 mol%. The modification amount of PVA6 was determined by measuring the n-DDM-modified vinyl ester-based polymer as a precursor before the saponification by ¹H-NMR.

**[Table 1]**

| | Polymer surfactant | Polymerization conditions | | | | Saponification conditions | | Physical properties | | | |
|---|---|---|---|---|---|---|---|---|---|---|---|
| | | Vinyl acetate | Methanol | Modifier | Polymerization rate | PVAc concentration | NaOH molar ratio | Degree of polymerization | Degree of saponification | Modification amount | HLB value |
| | | % by mass | % by mass | Type | % | % by mass | mol% | - | mol% | mol% | - |
| Production Example 1 | PVA1 | 40 | 60 | - | 50 | 30 | 0.005 | 530 | 88.0 | - | 15.8 |
| Production Example 2 | PVA2 | 40 | 60 | - | 50 | 30 | 0.004 | 530 | 72.0 | - | 11.4 |
| Production Example 3 | PVA3 | 40 | 60 | - | 50 | 30 | 0.020 | 530 | 98.5 | - | 19.4 |
| Production Example 4 | PVA4 | 70 | 30 | - | 30 | 30 | 0.005 | 1700 | 88.0 | - | 15.8 |
| Production Example 5 | PVA5 | 60 | 40 | MAPTAC *1) | 30 | 20 | 0.006 | 1200 | 88.0 | 2.0 | 16.7 |
| Production Example 6 | PVA6 | 60 | 40 | n-DDM *2) | 50 | 30 | 0.005 | 250 | 88.0 | 0.5 | 15.6 |

| | | | | | | | | | | | |
|---|---|---|---|---|---|---|---|---|---|---|---|
| *1) 3-(Methacryloylamino)propyltrimethylammonium chloride *2) Normal dodecyl mercaptan | | | | | | | | | | | |

### <Example 1>

### [Foam separation treatment test of PFAS]

A #240 ceramic cylindrical air stone having a diameter of 10 mm and a length of 150 mm was set on the bottom of a 1 L beaker, and 200 ml of an aqueous solution containing 1.0 ppm of PFOA (perfluorooctanoic acid) as PFAS and 100 ppm of PVA1 was charged thereto as water to be treated. Next, air was caused to flow through the air stone at a flow rate of 0.15 ml/min, and the air was fed into the water to be treated, and the generated foam was collected as needed for 30 minutes. The concentration of PFOA contained in the residual liquid after the test was analyzed using LC/MS(LC/MS-8030 manufactured by Shimadzu Corporation, column: TSKgel ODS-80Ts, detector: MS detector, ionization method: ESI mode, detected charge: Negative charge), and it was found to be 0.08 ppm.

### <Examples 2 to 10>

A separation test of PFAS was conducted under the same conditions as in Example 1 except that the types and concentrations of PVA and PFAS were changed as shown in Table 2. The results are shown in Table 2.

### <Example 11>

A separation test of PFAS was conducted under the same conditions as in Example 1 except that polyoxyethylene lauryl ether ("NONION K-230" manufactured by NOF Corporation, number of moles of ethylene oxide added: n = 30, HLB value: 17.5) was used instead of PVA1. The content of PFOA contained in the residual liquid after the test was 0.12 ppm. The results are shown in Table 2.

### <Comparative Example 1>

When a foam separation treatment test of PFAS was conducted under the same conditions as in Example 1 without addition of a surfactant, the content of PFOA contained in the remaining solution was 0.82 ppm.

### <Comparative Example 2>

A separation test of PFAS was conducted under the same conditions as in Example 1 except that lauryldimethylaminoacetic acid betaine ("NISSAN ANON BL-SF" manufactured by NOF Corporation, molecular weight: 271.44) was used instead of PVA 1. The content of PFOA contained in the residual liquid after the test was 0.75 ppm. The results are shown in Table 2.

**[Table 2]**

| | Surfactant | Foam separation treatment test conditions | | | | Foam separation treatment result | |
|---|---|---|---|---|---|---|---|
| | | PFAS type *3) | PFAS concentration | PVA concentration | PFAS content | PFAS concentration | PFAS residual ratio |
| | | | ppm | ppm | parts by mass *4) | ppm | % |
| Example 1 | PVA1 | PFOA | 1.0 | 100 | 1.0 | 0.08 | 8 |
| Example 2 | PVA2 | PFOA | 1.0 | 100 | 1.0 | 0.05 | 5 |
| Example 3 | PVA3 | PFOA | 1.0 | 100 | 1.0 | 0.20 | 20 |
| Example 4 | PVA4 | PFOA | 1.0 | 100 | 1.0 | 0.07 | 7 |
| Example 5 | PVA1 | PFOA | 0.1 | 100 | 0.1 | 0.005 | 5 |
| Example 6 | PVA1 | PFOA | 0.1 | 0.1 | 100 | 0.05 | 50 |
| Example 7 | PVA5 | PFOA | 1.0 | 100 | 1.0 | 0.06 | 6 |
| Example 8 | PVA6 | PFOA | 1.0 | 100 | 1.0 | 0.05 | 5 |
| Example 9 | PVA1 | PFHxS | 1.0 | 100 | 1.0 | 0.21 | 21 |
| Example 10 | PVA1 | PFBS | 1.0 | 100 | 1.0 | 0.60 | 60 |
| Example 11 | K-230 *1) | PFOA | 1.0 | 100 | 1.0 | 0.12 | 12 |
| Comparative Example 1 | - | PFOA | 1.0 | 0 | - | 0.82 | 82 |
| Comparative Example 2 | BL-SF *2) | PFOA | 1.0 | 100 | 1.0 | 0.75 | 75 |

| | | | | | | | |
|---|---|---|---|---|---|---|---|
| *1) "NONION K-230": polyoxyethylene lauryl ether (n = 30) *2) "NISSAN ANON BL-SF": lauryldimethylaminoacetic acid betaine *3) PFOA: perfluorooctanoic acid, PFHxS: perfluorohexanesulfonic acid, PFBS: perfluorobutanesulfonic acid *4) Content of PFAS based on 100 parts by mass of PVA | | | | | | | |

The results in Table 2 have shown that the PFAS residual ratio in Examples, in which the foam separation treatment was conducted using a polymer surfactant, was lower than the PFAS residual ratio in Comparative Examples, in which the treatment was conducted without using a polymer surfactant, and Examples are superior in separation and recovery efficiency.

## Claims

1. A foam separation treatment method of separating an organofluorine compound from water to be treated by collecting a foam generated by feeding a gas into the water to be treated containing the organofluorine compound, wherein
the foam separation treatment method comprises introducing a polymer surfactant into the water to be treated.

2. The foam separation treatment method according to claim 1, wherein the polymer surfactant is a vinyl alcohol-based polymer.

3. The foam separation treatment method according to claim 2, wherein the vinyl alcohol-based polymer has a viscosity-average degree of polymerization of 100 or more and 5,000 or less and a degree of saponification of 30 mol% or more and 99.99 mol% or less.

4. The foam separation treatment method according to claim 1, wherein the polymer surfactant is introduced into the water to be treated such that a content of the polymer surfactant in the water to be treated is 1% by mass or less.

5. The foam separation treatment method according to any one of claims 1 to 4, wherein the polymer surfactant is introduced in an amount of 1 or more times by mass an amount of the organofluorine compound contained in the water to be treated.

6. An aqueous solution comprising a vinyl alcohol-based polymer and an organofluorine compound.

7. The aqueous solution according to claim 6, wherein the vinyl alcohol-based polymer has a viscosity-average degree of polymerization of 100 or more and 5,000 or less and a degree of saponification of 30 mol% or more and 99.99 mol% or less.

8. The aqueous solution according to claim 6, wherein a content of the vinyl alcohol-based polymer in the aqueous solution is 1% by mass or less.

9. The aqueous solution according to claim 6, wherein a content of the vinyl alcohol-based polymer is 1 or more times by mass a content of the organofluorine compound in the aqueous solution.

10. A foam separation treatment method comprising: feeding a gas into the aqueous solution according to any one of claims 6 to 9 to generate a foam; and collecting the foam.

11. A method of using a vinyl alcohol-based polymer in a treatment of separating an organofluorine compound from water to be treated by collecting a foam generated by feeding a gas into the water to be treated containing the organofluorine compound,
the method comprising mixing a vinyl alcohol-based polymer with the water to be treated to promote generation of the foam.

12. A foam generation promoter comprising a vinyl alcohol-based polymer to be introduced into water to be treated when an organofluorine compound is separated from the water to be treated by a foam separation treatment.
